# EUROPEAN PATENT APPLICATION

(11) **EP 4 847 006 A1**
(43) Date of publication of application: **02.12.2026**
(21) Application number: 25179836.9
(22) Date of filing: 30.05.2025
(51) Int. Cl.: A61K 8/34, A61K 8/41, A61K 8/49, A61K 8/67, A61Q 5/10

(54) **OXIDATIVE HAIR DYE COMPLEX, COMPOSITION, METHOD AND USE**

(71) Applicant: Kao Corporation, Chuo-ku, Tokyo 103-8210 (JP)
(72) Inventor: MÖHRING, Hartmut, 64297 Darmstadt (DE); SHIMIZU, Kohei, Chuo-ku, Tokyo, 103-8210 (JP); FUKUHARA, Yoshiki, Chuo-ku, Tokyo, 103-8210 (JP)
(74) Representative: Hoffmann Eitle

(57) **Abstract**

An oxidative hair dye complex comprising one or more compound(s) according to group X and one or more compound(s) according to group Y, preferably the one or more compound(s) according to group X are selected from thiamine, thiamine monophosphate, thiamine pyrophosphate, thiamine triphosphate, adenosine thiamine diphosphate, adenosine thiamine triphosphate, hydrochloride, and preferably the compound(s) according to group Y are selected from resorcinol, 2-methylresorcinol, 4-chlororesorcinol, 2-amino-3-hydroxypyridine, 5-amino-6-chloro-o-cresol, 1,3-bis(2,4-diamino-phenoxy)-propane, and/or their salt(s), and/or their mixtures.

## Description

### Field of the invention

The present invention relates to an oxidative dye complex, and oxidative dyeing composition, method of making the complex, method of dyeing, and use of compound.

### Background of the invention

Many people desire hair colors that are different from their natural hair color. There are at least three classes of compounds commonly used for hair coloring: 1) oxidative dyes, 2) direct dyes, and 3) pigments. In oxidative dyeing, colorless molecules are applied to the hair, penetrate the hair fiber due to their small molecular weight, and then polymerize in the presence of an oxidizing agent to form larger molecules that cannot be easily washed out. Direct dyes are colored compounds that are applied to the hair and color the hair according to its inherent color. Pigments are water-insoluble compounds that are commonly used in temporary hair coloration because they are deposited on the hair surface and are easily washed off.

There are many known product forms of oxidative hair dyes, the majority of which are aqueous. Like any other industry, the cosmetics industry is developing more and more products with improved or equivalent performance, but with a smaller environmental footprint. In recent years, solid formulations of oxidative hair dyes have received increasing attention because they are water-depleted, allow for a reduced product weight and therefore result in less carbon dioxide emissions during production, packaging, storage, and shipping.

One of the challenges with solid oxidative dye blends is their dyeing intensity after storage. The oxidative dyes tend to polymerize prematurely during storage, resulting in a reduced coloring effect on the consumer's hair.

Therefore, oxidative dyes need to be stabilized to prolong their storage stability, especially in water-depleted product forms. Several stabilization approaches are known, but there is still a need for better solutions.

### Summary of the invention

Therefore, the first object of the present invention is an oxidative hair dye complex comprising one or more compound(s) according to group X and one or more compound(s) according to group Y,
wherein the compound(s) according to group X have the following general structure: 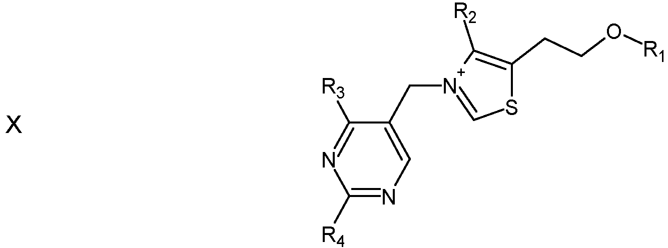
wherein R₁ represents a hydrogen atom, a phosphonate group, a pyrophosphate group, a triphosphate group, an adenosine diphosphate group, an adenosine triphosphate group, a linear or branched C₁-C₈ alkyl group, a linear or branched C₂-C₈ alkenyl group,
and wherein R₂, R₃, and R₄ represent independently a hydrogen, an amino group, linear or branched C₁-C₆ alkyl which may be substituted with one hydroxyl group, or linear or branched C₃-C₁₂ alkenyl which may be substituted with one hydroxyl group, wherein at least one of R₂, R₃, or R₄ is different from H,
and/or their salts, and/or their mixtures,
preferably the one or more compound(s) according to group X are selected from thiamine, thiamine monophosphate, thiamine pyrophosphate, thiamine triphosphate, adenosine thiamine diphosphate, adenosine thiamine triphosphate, and/or their salt(s),
and wherein the compound(s) according to group Y are selected from the following general structures: 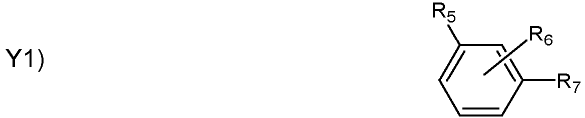
wherein R₅ and R₇ represent independently a hydroxyl group, an amino group, a sulfhydryl group, a linear or branched C₁-C₆ alkyl or alkenyl group, a halogen group, R₆ represents a hydrogen, a hydroxyl group, a halogen group, or a linear or branched C₁-C₆ alkyl or alkenyl group,
preferably the one or more compound(s) according to group Y1 are selected from resorcinol, 2-methylresorcinol, 4-chlororesorcinol, 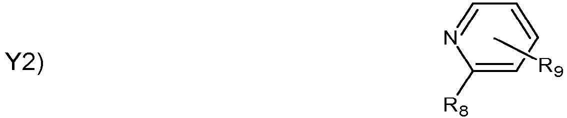
wherein R₈ and R₉ represent independently a hydroxyl group, an amino group, a sulfhydryl group, a linear or branched C₁-C₆ alkyl or alkenyl group, a halogen group,
preferably the one or more compound(s) according to group Y2 are selected from 2-amino-3-hydroxypyridine, 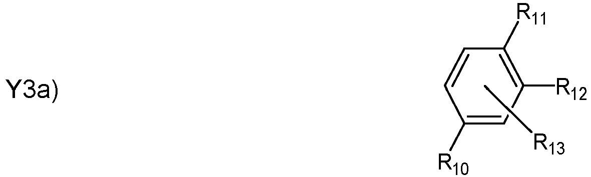 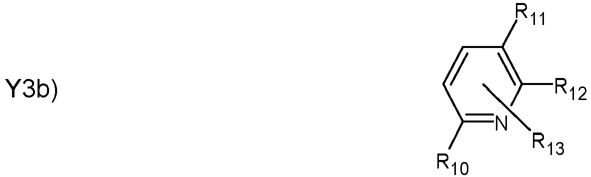
wherein R₁₀, R₁₁, R₁₂, and R₁₃ represent independently a hydrogen group, a hydroxyl group, an amino group, a methoxy group, a sulfhydryl group, a linear or branched C₁-C₆ alkyl or alkenyl group, a halogen group,
preferably the one or more compound(s) according to group Y3a or Y3b are selected from 5-amino-6-chloro-o-cresol, 2,6-dihydroxy-3,4-dimethyl-pyridine, 2,6-dimethoxy-3,5-pyridine-diamine HCl, 5-amino-2,6-dimethoxy-3-hydroxypyridine, 6-methoxy-2-methylamino-3-aminopyridine HCl, 2,3-diamino-6-methoxy-pyridine, 2,6-diaminopyridine, and/or their salt(s), 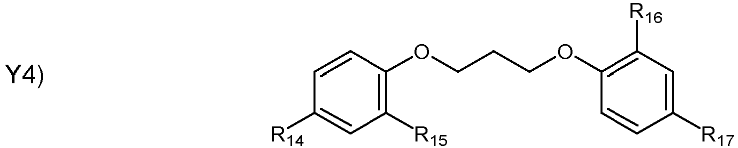
wherein R₁₄, R₁₅, R₁₆, and R₁₇ represent independently a hydroxyl group, an amino group, a sulfhydryl group, a linear or branched C₁-C₆ alkyl or alkenyl group, a halogen group, and/or their salt(s), and/or their mixtures,
preferably the one or more compound(s) according to group Y4 are selected from 1,3-bis(2,4-diamino-phenoxy)-propane.

The second object of the present invention is an oxidative hair dyeing composition comprising the complex as defined above, wherein the oxidative hair dyeing composition comprises water at 10% by weight or less, calculated to the total weight of the oxidative hair dyeing composition.

The third object of the present invention is a method of making the complex as defined above comprising the steps of:
a) dissolving the one or more compounds X and Y in a solvent, preferably in water, followed by
b) evaporating the solvent and drying the complex.

The fourth object of the present invention is a kit-of-parts comprising:
- the composition as defined above, and
- a second aqueous composition having a pH in the range of 1 to 6 and comprising one or more oxidizing agent(s), preferably hydrogen peroxide.

The fifth object of the present invention is a method for dyeing keratin fibers, preferably human keratin fibers, more preferably human hair, comprising the steps of:
(i) mixing the composition as defined above with a second aqueous composition as defined above to yield a ready-to-use composition having a pH in the range of 6 to 12,
(ii) applying the ready-to-use composition onto keratin fibers and leaving it for a time period in the range of 1 min to 60 min,
(iii) rinsing-off the keratin fibers and optionally shampooing the keratin fibers.

The sixth object of the present invention is the use of the compound X and/or its salt(s) as defined above for improving the storage stability of the compound(s) Y and/or their salt(s) as defined above in oxidative hair dyeing compositions comprising water at 10% by weight or less, calculated to the total weight of the oxidative hair dyeing composition.

### Detailed description of the invention

The inventors of the present invention have surprisingly and unexpectedly found that the complex, the composition, the kit, and the methods according to the independent claims improved the dyeing intensity after storage of the oxidative dyes, increased sustainability, and increased consumer satisfaction, while conferring the hair good cosmetic properties such as feel and touch.

### Oxidative hair dye complex

The present invention is directed to an oxidative hair dye complex comprising one or more compound(s) according to group X and one or more compound(s) according to group Y,
wherein the compound(s) according to group X have the following general structure: 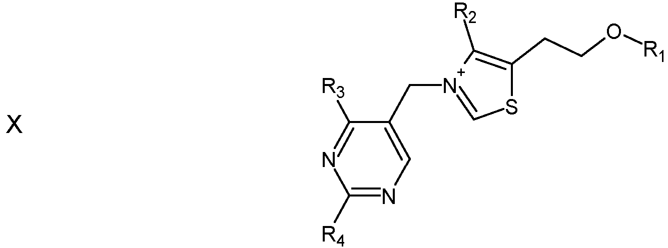
wherein R₁ represents a hydrogen atom, a phosphonate group, a pyrophosphate group, a triphosphate group, an adenosine diphosphate group, an adenosine triphosphate group, a linear or branched C₁-C₈ alkyl group, a linear or branched C₂-C₈ alkenyl group,
and wherein R₂, R₃, and R₄ represent independently a hydrogen, an amino group, linear or branched C₁-C₆ alkyl which may be substituted with one hydroxyl group, or linear or branched C₃-C₁₂ alkenyl which may be substituted with one hydroxyl group, wherein at least one of R₂, R₃, or R₄ is different from H, and/or their salts, and/or their mixtures,
preferably the one or more compound(s) according to group X are selected from thiamine, thiamine monophosphate, thiamine pyrophosphate, thiamine triphosphate, adenosine thiamine diphosphate, adenosine thiamine triphosphate, and/or their salt(s),
and wherein the compound(s) according to group Y are selected from the following general structures: 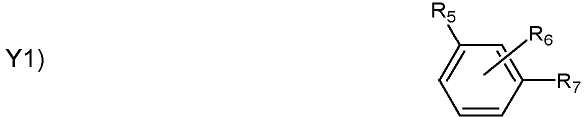
wherein R₅ and R₇ represent independently a hydroxyl group, an amino group, a sulfhydryl group, a linear or branched C₁-C₆ alkyl or alkenyl group, a halogen group, R₆ represents a hydrogen, a hydroxyl group, a halogen group, or a linear or branched C₁-C₆ alkyl or alkenyl group,
preferably the one or more compound(s) according to group Y1 are selected from resorcinol, 2-methylresorcinol, 4-chlororesorcinol, 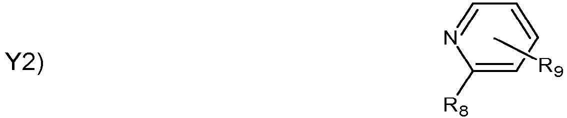
wherein R₈ and R₉ represent independently a hydroxyl group, an amino group, a sulfhydryl group, a linear or branched C₁-C₆ alkyl or alkenyl group, a halogen group,
preferably the one or more compound(s) according to group Y2 are selected from 2-amino-3-hydroxypyridine, 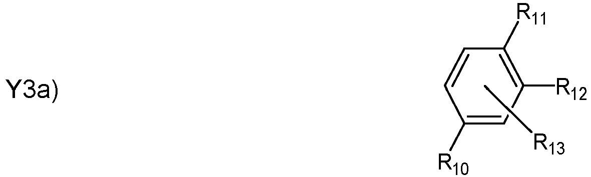 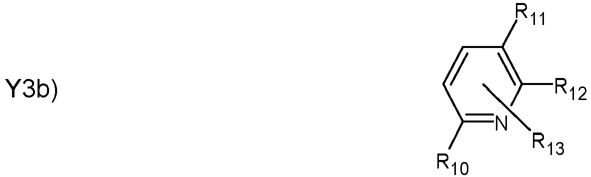
wherein R₁₀, R₁₁, R₁₂, and R₁₃ represent independently a hydrogen group, a hydroxyl group, an amino group, a methoxy group, a sulfhydryl group, a linear or branched C₁-C₆ alkyl or alkenyl group, a halogen group,
preferably the one or more compound(s) according to group Y3a or Y3b are selected from 5-amino-6-chloro-o-cresol, 2,6-dihydroxy-3,4-dimethyl-pyridine, 2,6-dimethoxy-3,5-pyridine-diamine HCl, 5-amino-2,6-dimethoxy-3-hydroxypyridine, 6-methoxy-2-methylamino-3-aminopyridine HCl, 2,3-diamino-6-methoxy-pyridine, 2,6-diaminopyridine, and/or their salt(s), 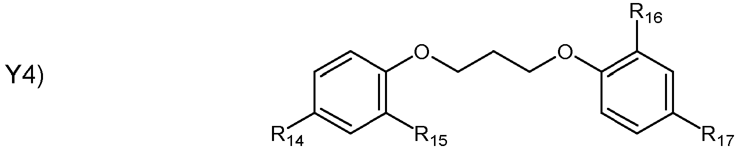
wherein R₁₄, R₁₅, R₁₆, and R₁₇ represent independently a hydroxyl group, an amino group, a sulfhydryl group, a linear or branched C₁-C₆ alkyl or alkenyl group, a halogen group, and/or their salt(s), and/or their mixtures.

Preferably the one or more compound(s) according to group Y4 are selected from 1,3-bis(2,4-diamino-phenoxy)-propane, and/or their salts.

Suitable counterions for salts of compounds X and Y are hydrochloride, hydrobromide, chloride, bromide, methosulphate, sulphate, and/or their mixtures.

It is preferred that the molar ratio of one or more compound(s) according to group X to one or more compound(s) according to group Y as defined above is in the range of 0.8 to 1.2, preferably in the range of 0.9 to 1.1, more preferably 1.0, calculated to the free base of each of the compound(s). Thus, the molar ratio is formed by neglecting the molar masses of the counterion salts.

Suitably, the complex is formed with thiamine hydrochloride as compound X and with one or more compound(s) according to group Y selected from 4-chlororesorcinol, 1,3-bis(2,4-diamino-phenoxy)-propane, 2-methylresrocinol, 5-amino-6-chloro-o-cresol, and/or 2-amino-3-hydroxypyridine, and/or their salt(s), and/or their mixtures.

Suitably, the complex is formed with thiamine as compound X and with one or more compound(s) according to group Y selected from 4-chlororesorcinol, 1,3-bis(2,4-diamino-phenoxy)-propane, 2-methylresrocinol, 5-amino-6-chloro-o-cresol, and/or 2-amino-3-hydroxypyridine, and/or their salt(s), and/or their mixtures and makes a particle.

### Oxidative Hair Dyeing Composition

The present invention is also directed to an oxidative hair dyeing composition comprising the complex as defined above, wherein the oxidative hair dyeing composition comprises water at 10% by weight or less, calculated to the total weight of the oxidative hair dyeing composition.

The water content of the composition may be determined by the methods well known in the art. For example, the water content may be determined by Karl-Fischer-Titration.

It is preferred that the total concentration by weight of the complex as defined above in the oxidative hair dyeing composition is in the range of 0.1% to 90% by weight, more preferably in the range of 0.25% to 80% by weight, still more preferably in the range of 0.5% to 70% by weight, still more preferably in the range of 0.5% to 65% by weight, still more preferably in the range of 0.5% to 50% by weight, still more preferably in the range of 0.5% to 20% by weight, and still more preferably in the range of 0.5% to 10% by weight, calculated to the total weight of the oxidative hair dyeing composition.

It is preferred that the oxidative hair dyeing composition as defined above comprises one or more pulverulent excipients. The term 'excipient' refers to compounds which do not chemically react with any ingredient of the oxidative dyeing composition. Such compounds may be seen as bulking agents, as they disperse the ingredients of the oxidative dyeing composition.

Preferably, such pulverulent excipients are selected from organic/inorganic materials such as diatomaceous earth, cellulose, starch especially corn, tapioca, rice, wheat and potato, nylon powder, gypsum, sawdust and perlite, more preferably said pulverulent excipient is cornstarch.

It is preferred that the total concentration of one or more pulverulent excipients in the oxidative hair dyeing composition is in the range of 1% to 80% by weight, preferably in the range of 2% to 75% by weight, more preferably in the range of 2.5% to 70% by weight,-calculated to the total weight of the oxidative hair dyeing composition.

It is preferred that the total concentration of water in the oxidative hair dyeing composition as defined above is 10% by weight or less, more preferably 5% by weight or less, still more preferably 1% by weight or less, still more preferably 0.5% by weight or less, calculated to the total weight of oxidative hair dyeing composition, most preferably the oxidative hair dyeing composition is anhydrous.

It is preferred that the oxidative hair dyeing composition as defined above is a hair dyeing powder, a hair dyeing tablet, or a hair dyeing pellet.

Preferably, the oxidative hair dyeing composition comprises one or more lipophilic compounds(s) being liquid at 25°C and atmospheric pressure.

Suitable non-limiting examples of the one or more lipophilic compound(s) is/are isopropyl myristate (IPM), paraffin, squalane, phenyl trimethicone, and silicone, and/or their mixtures.

It is preferred that the total concentration of the lipophilic compound(s) as defined above in the oxidative hair dyeing composition is in the range of 0.1% to 20% by weight, more preferably in the range of 1% to 10% by weight, most preferably in the range of 2% to 6% by weight, calculated to the total weight of the oxidative hair dyeing composition.

In another aspect of the present invention, preferably the oxidative hair dyeing composition is a hair dyeing oil and the total concentration of lipophilic compounds as defined above is in the range of 20% to 50% by weight, more preferably in the range of 25% to 40% by weight, most preferably in the range of 25% to 30% by weight, calculated to the total weight of the composition.

### Alkalizing agent(s)

It is preferred that the oxidative hair dyeing composition as defined above comprises one or more alkalizing agent(s).

Suitable non-limiting examples of the one or more alkalizing agent(s) is/are one or more phosphate(s), one or more disilicate(s), one or more metasilicate(s), one or more carbonate(s), one or more bicarbonate(s), one or more Tris(hydroxymethyl)aminomethane (TRIS), silicate(s), and/or its/their salts, and/or their mixtures, preferably their alkali and earth alkali salts.

Preferably said one or more alkalizing agent(s) is/are one or more phosphate(s) or one or more metasilicate(s), and/or its/their salts, and/or their mixtures.

The most preferred alkalizing agents are trisodium or tripotassium phosphate and sodium metasilicate, and/or their mixtures.

It is preferred that the total concentration of alkalizing agent(s) in the oxidative hair dyeing composition is in the range of 1% to 75% by weight, more preferably 5% to 70% by weight, still more preferably in the range of 10% to 70% by weight, still more preferably in the range of 15% to 70% by weight, calculated to the total weight of the oxidative hair dyeing composition.

Suitably, the alkalizing agents are further coated with lipophilic agents, such as C₁₂-C₂₂ fatty alcohols or esters. The lipophilic coating reduces water-adsorption of the alkalizing agent.

### Compound(s) according to group Z

It is preferred that the oxidative hair dyeing composition as defined above comprises one or more compound(s) according to group Z having the following general structure 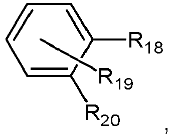 wherein R₁₈, R₁₉, and R₂₀ represent independently a hydrogen group, a hydroxyl group, an amino group, a sulfhydryl group, a methyl group, chlorine group, an acyl chloride group, C₁-C₄ alkoxy, C₁-C₄ hydroxyalkyl, preferably the one or more compound(s) according to group Z are selected from o-phenylenediamines, p-phenylenediamines, more preferably the one or more compound(s) according to group Z is p-phenylenediamine, 2-methoxy-methyl-p-phenylenediamine, p-toluene-diamine, hydroxyethyl-p-phenylene-diamine, and/or their salt(s), and/or their mixtures.

The preferred one or more compound(s) according to group Z is 2-methoxy-methyl-p-phenylene-diamine, and/or its salt, most preferably it is 2-methoxy-methyl-p-phenylene-diamine sulfate.

It is preferred that the total concentration of the one or more compound(s) according to group Z as defined above in the oxidative hair dyeing composition is in the range of 0.05% to 10% by weight, more preferably in the range of 0.1% to 7% by weight, most preferably in the range of 0.15% to 5% by weight, calculated to the total weight of the oxidative hair dyeing composition.

### Compound(s) according to group Y

Suitable one or more compound(s) according to group Y are selected from 4-chlororesorcinol, 1,3-bis(2,4-diamino-phenoxy)-propane, 2-methylresrocinol, 5-amino-6-chloro-o-cresol, and/or 2-amino-3-hydroxypyridine, and/or their salt(s), and/or their mixtures.

It is preferred that the complex is formed with thiamine hydrochloride as compound X and with one or more compound(s) according to group Y selected from 4-chlororesorcinol, 1,3-bis(2,4-diamino-phenoxy)-propane, 2-methylresrocinol, 5-amino-6-chloro-o-cresol, and/or 2-amino-3-hydroxypyridine, and/or their salt(s), and/or their mixtures.

It is preferred that the complex is formed with thiamine as compound X and with one or more compound(s) according to group Y selected from 4-chlororesorcinol, 1,3-bis(2,4-diamino-phenoxy)-propane, 2-methylresrocinol, 5-amino-6-chloro-o-cresol, and/or 2-amino-3-hydroxypyridine, and/or their salt(s), and/or their mixtures.

It is preferred that the molar ratio of compound(s) according to group Y and the compound(s) according to group Z is in the range of 0.8 to 1.2, more preferably in the range of 0.9 to 1.1, most preferably it is 1.0, calculated to the free base of each of the compound(s).

It is preferred that the total concentration of one or more compound(s) according to group Y as defined above complexed by the one or more compound(s) according to group X, preferably said compound according to group X is thiamine or thiamine hydrochloride, in the oxidative hair dyeing composition is in the range of 0.1% to 90% by weight, preferably in the range of 0.25% to 80% by weight, more preferably in the range of 0.5% to 70% by weight, still more preferably in the range of 0.5% to 65% by weight, still more preferably in the range of 0.5% to 50% by weight, still more preferably in the range of 0.5% to 20% by weight, and still more preferably in the range of 0.5% to 10% by weight, calculated to the total weight of the oxidative hair dyeing composition.

### Surfactants

Optionally, the oxidative dyeing composition according to the present invention may further comprise one or more surfactant(s), preferably selected from non-ionic surfactants, anionic surfactants, cationic surfactants, and/or amphoteric/zwitterionic surfactants, and/or their salt(s), and/or their mixtures, more preferably selected from anionic surfactants and/or their salt(s), from the viewpoint of improving wettability and mixability.

Preferably, the anionic surfactants may be selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures, and/or their salts.

Suitable examples are alkyl sulfate or preferably ethoxylated alkyl ether sulfate surfactants or mixtures thereof, and/or salts thereof, having an alkyl chain length of C₁₀ to C₂₂ and an ethoxylation degree from 1 to 50.

Suitable non-ionic surfactants may be selected from alkyl polyglycosides, ethoxylated triglycerides, ethoxylated fatty alcohols, ethoxylated fatty acid esters, and/or their mixtures.

Suitable cationic surfactants are quaternary ammonium surfactants having a carbon chain length in the range of C₁₂ to C₂₂ or surfactants having a tertiary amine group and at least one alkyl chain having a carbon chain length in the range of C₁₂ to C₂₂ such as alkylamidoalkylamine surfactants, and/or their salts. Suitable examples are cetrimonium chloride and behentrimonium chloride.

Suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine. The disclosure also relates to the salts of the compounds.

Suitable concentration ranges for surfactants are in the range of 0.1% to 10% by weight, calculated to the total weight of the oxidative hair dyeing composition, from the viewpoint of enhancing wettability of keratin fibers, and mixability with other compositions.

### Thickening polymers

From the viewpoint of cosmetic safety, it is further preferred that the oxidative hair dyeing composition comprises one or more thickening polymer(s).

The oxidative hair dyeing composition may comprise one or more thickening polymer(s) selected from non-ionic thickening polymers and/or anionic thickening polymers, and/or their mixtures.

Preferably, the thickening polymers are selected from polymers resulting in an aqueous solution and/or aqueous dispersion at pH ranges between 7 and 12 having a viscosity of at least 1,000 mPa•s measured at a polymer concentration of 1% by weight in water at 25°C determined by cone plate viscometry at 25°C under atmospheric conditions, calculated to the total weight of the composition, determined by cone-plate viscometry, suitably with a Brookfield viscometer with spindle #4 measured at 10 rpm for 1 min at 25°C and atmospheric conditions.

Suitable non-ionic thickening polymers are cellulose-based polymers. Suitable examples of cellulose-based polymers are methylcellulose, hydroxymethylcellulose, hydroxyethylcellulose, hydroxypropylcellulose, hydroxyethyl-methylcellulose, and alkylated hydroxyl celluloses such as (C₂-C₈)-alkylcelluloses or cetyl hydroxyethylcellulose.

Suitable anionic thickening polymers are selected from naturally-based anionic polymers and/or synthetic anionic polymers.

Suitably, the natural anionic polymer(s) may be selected from xanthan gum, dehydroxanthan gum, hydroxypropylxanthan gum, carboxymethyl cellulose and starch-based polymers such as vegetable starch and/or their synthetically modified derivatives such as hydroxypropyl starch phosphate. Equally suitable are alginic acids, sodium alginates, ammonium alginates, calcium alginates, gum arabic, and guar gum.

Suitable synthetic anionic polymers are associative thickening polymers, such as acrylates/steareth-30 methacrylate copolymer.

The preferred thickening polymer for the composition of the present invention are natural anionic polymers, more preferably xanthan gum and/or dehydroxanthan gum, from the viewpoint of their biodegradability and low environmental impact.

Preferably, the total concentration of thickening polymers of the present invention are 0.1% by weight or more, more preferably 0.25% by weight or more, more preferably 0.5% by weight or more, calculated to the total weight of the oxidative hair dyeing composition, from the viewpoint of providing sufficient viscosity to the oxidative hair dyeing composition upon contact with water.

Preferably, the total concentration of thickening polymers in the oxidative hair dyeing composition of the present invention are 15% by weight or less, more preferably 12% by weight or more, further more preferably 10% by weight or less, calculated to the total weight of the oxidative hair dyeing composition, from the viewpoint of providing sufficient viscosity to the composition upon contact with water and cost of goods.

For attaining the above-mentioned effects, it is preferred that the total concentration of thickening polymers in the oxidative hair dyeing composition of the present invention is in the range of 0.1% to 15% by weight, preferably in the range of 0.25% to 12% by weight, more preferably in the range of 0.5% to 10% by weight, calculated to the total weight of the oxidative hair dyeing composition.

### Method of making the complex

A method of making the complex as defined above comprising the steps of:
a) dissolving the one or more compounds X and Y in a solvent, preferably in water, followed by
b) evaporating the solvent and drying the complex.

Preferably, for evaporating in step b) heat is applied to the complex in the temperature range of 30°C to 100°C, more preferably in the temperature range of 30°C to 80°C, still more preferably in the range of 50°C to 80°C.

### Kit-of-parts

The present invention is also directed to a kit-of-parts comprising:
- the oxidative hair dyeing composition as defined above, and
- a second aqueous composition having a pH in the range of 1 to 6 and comprising one or more oxidizing agent(s), preferably hydrogen peroxide.

Preferably the total concentration of the hydrogen peroxide is in the range of 1% to 20% by weight, more preferably in the range of 2% to 15% by weight, most preferably in the range of 3% to 12% by weight, calculated to the total weight of the second aqueous composition.

It is preferred that the pH of the second aqueous composition is in the range of 1 to 5, more preferably the pH is in the range of 2 to 5, more preferably the pH is in the range of 2.5 to 4.5.

It is further preferred that the oxidative dyeing composition as defined above and the second aqueous composition are kept separate until directly prior to the application onto keratin fibers.

A preferred mixing ratio by weight of the oxidative hair dyeing composition and the second aqueous composition is in the range of 0.1 to 2.

Preferably, from the viewpoint of cosmetic safety, the mixture of the oxidative hair dyeing composition and second aqueous composition has a viscosity in the range of 1,000 Pas to 25,000 mPas, preferably 2,000 mPas to 20,000 mPas, more preferably in the range of 2,500 mPas to 17,500 mPas, determined by cone plate viscometry at 25°C under atmospheric conditions. A method for determining the viscometry is cone-plate viscometry, suitably with a Brookfield viscometer and spindle #4, measured at 10 rpm for 1 min at 25°C.

### Method for dyeing keratin fibers

The present invention is also directed to a method for dyeing keratin fibers, preferably human keratin fibers, more preferably human hair, comprising the steps of:
(i) mixing the oxidative dyeing composition as defined above with a second aqueous composition as defined above to yield a ready-to-use composition having a pH in the range of 6 to 12,
(ii) applying the ready-to-use composition onto keratin fibers and leaving it for a time period in the range of 1 min to 60 min,
(iii) rinsing-off the keratin fibers and optionally shampooing the keratin fibers.

Preferably the pH of the ready-to-use composition in step (i) is in the range of 7 to 11, more preferably in the range of 8 to 11, furthermore preferably in the range of 8.5 to 10.5.

Further, it is preferred that the ready-to-use composition is left in step (ii) onto keratin fibers for a time period in the range of 5 to 45 min, more preferably 10 to 40 min.

It is also preferred to set the temperature of the keratin fibers during the leaving step ii) in the range of 20°C to 40°C.

### Use of the compound X

The present invention is also directed to a use of the compound X and/or its salt(s) as defined above for improving the storage stability of the compound(s) Y and/or their salt(s) as defined above in oxidative hair dyeing composition comprising water at 10% by weight or less, calculated to the total weight of the oxidative hair dyeing composition.

The present invention is also directed to <1> an oxidative hair dye complex comprising one or more compound(s) according to group X and one or more compound(s) according to group Y,
wherein the compound(s) according to group X have the following general structure: 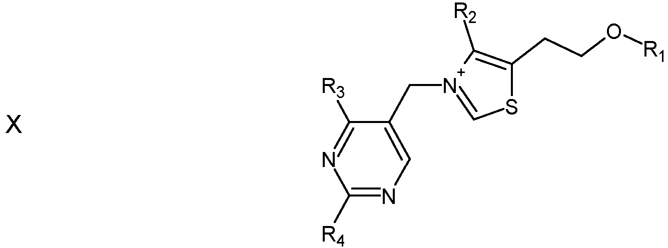
wherein R₁ represents a hydrogen atom, a phosphonate (phosphonic acid) group, a pyrophosphate (diphosphate) group, a triphosphate group, an adenosine diphosphate group, an adenosine triphosphate group, a linear or branched C₁-C₈ alkyl group, a linear or branched C₂-C₈ alkenyl group,
and wherein R₂, R₃, and R₄ represent independently a hydrogen, an amino group, linear or branched C₁-C₆ alkyl which may be substituted with one hydroxyl group, or linear or branched C₃-C₁₂ alkenyl which may be substituted with one hydroxyl group, wherein at least one of R₂, R₃, or R₄ is different from H,
and/or their salts, and/or their mixtures,
preferably the one or more compound(s) according to group X are selected from thiamine, thiamine monophosphate, thiamine pyrophosphate, thiamine triphosphate, adenosine thiamine diphosphate, adenosine thiamine triphosphate, and/or their salt(s),
and wherein the compound(s) according to group Y are selected from the following general structures: 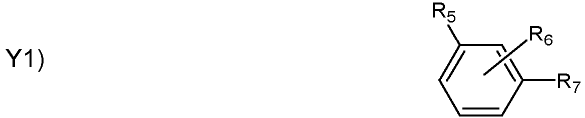
wherein R₅ and R₇ represent independently a hydroxyl group, an amino group, a sulfhydryl group, a linear or branched C₁-C₆ alkyl or alkenyl group, a halogen group, R₆ represents a hydrogen, a hydroxyl group, a halogen group, or a linear or branched C₁-C₆ alkyl or alkenyl group,
preferably the one or more compound(s) according to group Y1 are selected from resorcinol, 2-methylresorcinol, 4-chlororesorcinol, 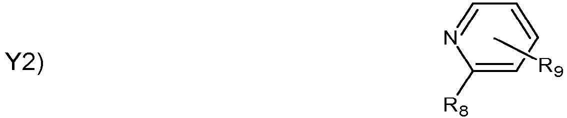
wherein R₈ and R₉ represent independently a hydroxyl group, an amino group, a sulfhydryl group, a linear or branched C₁-C₆ alkyl or alkenyl group, a halogen group,
preferably the one or more compound(s) according to group Y2 are selected from 2-amino-3-hydroxypyridine, 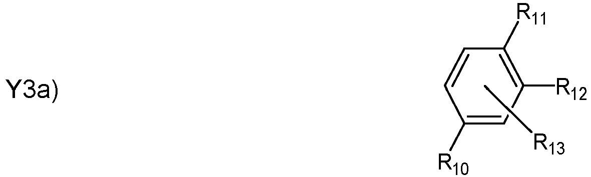 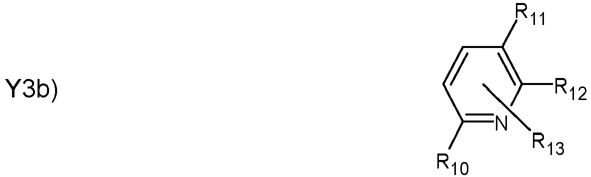
wherein R₁₀, R₁₁, R₁₂, and R₁₃ represent independently a hydrogen group, a hydroxyl group, an amino group, a methoxy group, a sulfhydryl group, a linear or branched C₁-C₆ alkyl or alkenyl group, a halogen group,
preferably the one or more compound(s) according to group Y3a or Y3b are selected from 5-amino-6-chloro-o-cresol, 2,6-dihydroxy-3,4-dimethyl-pyridine, 2,6-dimethoxy-3,5-pyridine-diamine HCl, 5-amino-2,6-dimethoxy-3-hydroxypyridine, 6-methoxy-2-methylamino-3-aminopyridine HCl, 2,3-diamino-6-methoxy-pyridine, 2,6-diaminopyridine, and/or their salt(s), 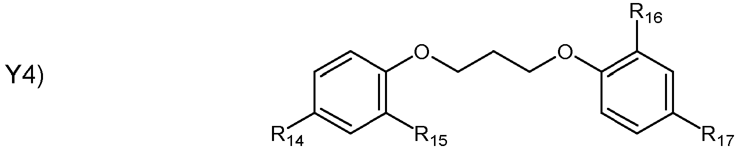
wherein R₁₄, R₁₅, R₁₆, and R₁₇ represent independently a hydroxyl group, an amino group, a sulfhydryl group, a linear or branched C₁-C₆ alkyl or alkenyl group, a halogen group, and/or their salt(s), and/or their mixtures,
preferably the one or more compound(s) according to group Y4 are selected from 1,3-bis(2,4-diamino-phenoxy)-propane.

<2> The complex according to clause <1> characterized in that the one or more compound(s) according to group X are selected from thiamine, and/or its salt(s), and one or more compound(s) according to group Y are selected from
Y1) resorcinol, 2-methylresorcinol, 4-chlororesorcinol,
Y2) 2-amino-3-hydroxypyridine,
Y3) 5-amino-6-chloro-o-cresol, 2,6-dihydroxy-3,4-dimethyl-pyridine, 2,6-dimethoxy-3,5-pyridine-diamine HCl, 5-amino-2,6-dimethoxy-3-hydroxypyridine, 6-methoxy-2-methylamino-3-aminopyridine HCl, 2,3-diamino-6-methoxy-pyridine, 2,6-diaminopyridine,
Y4) 1,3-bis(2,4-diamino-phenoxy)-propane, and/or their salt(s), and/or their mixtures.

<3> The complex according to any of the clauses <1> to <2> characterized in that the one or more compound(s) according to group X are selected from thiamine, and/or its salt(s), and one or more compound(s) according to group Y are selected from Y1) resorcinol, 2-methylresorcinol, 4-chlororesorcinol, and/or their salt(s), and/or their mixtures.

<4> The complex according to any of the clauses <1> to <2> characterized in that the one or more compound(s) according to group X are selected from thiamine, and/or its salt(s), and the one or more compound(s) according to group Y are selected from Y2) 2-amino-3-hydroxypyridine, and/or its salt(s).

<5> The complex according to any of the clauses <1> to <2> characterized in that the one or more compound(s) according to group X are selected from thiamine, and/or its salt(s), and the one or more compound(s) according to group Y are selected from Y3) 5-amino-6-chloro-o-cresol, and/or its salt(s).

<6> The complex according to any of the clauses <1> to <2> characterized in that the one or more compound(s) according to group X are selected from thiamine, and/or its salt(s), and one or more compound(s) according to group Y are selected from Y4) 1,3-bis(2,4-diamino-phenoxy)-propane, and/or its salt(s).

<7> The complex according to any of the clauses <1> to <2> characterized in that the one or more compound(s) according to group X are selected from thiamine hydrochloride, and the one or more compound(s) according to group Y are selected from Y1) resorcinol, 2-methylresorcinol, 4-chlororesorcinol, and/or their salt(s), and/or their mixtures.

<8> The complex according to any of the clauses <1> to <2> characterized in that the one or more compound(s) according to group X are selected from thiamine
hydrochloride, and the one or more compound(s) according to group Y are selected from Y2) 2-amino-3-hydroxypyridine, and/or its salt(s).

<9> The complex according to any of the clauses <1> to <2> characterized in that the one or more compound(s) according to group X are selected from thiamine hydrochloride and the one or more compound(s) according to group Y are selected from Y3) 5-amino-6-chloro-o-cresol, and/or its salt(s).

<10> The complex according to any of the clauses <1> to <2> characterized in that the one or more compound(s) according to group X are selected from thiamine hydrochloride and the one or more compound(s) according to group Y are selected from Y4) 1,3-bis(2,4-diamino-phenoxy)-propane, and/or its salt(s).

<11> The complex according any of the clauses <1> to <10> characterized in that the molar ratio of the one or more compound(s) according to group X to the one or more compound(s) according to group Y as defined above is in the range of 0.8 to 1.2, preferably in the range of 0.9 to 1.1, more preferably 1.0, calculated to the free base of each of the compound(s).

The present disclosure is also related to <12> an oxidative hair oxidative hair dyeing composition comprising the complex according to any of the clauses <1> to <11> wherein the oxidative hair dyeing composition comprises water at 10% by weight or less, calculated to the total weight of the oxidative hair dyeing composition.

<13> The composition according to clause <12> characterized in that the total concentration by weight of the complex according to any of the clauses <1> to <11> in the oxidative hair dyeing composition is in the range of 0.1% to 90% by weight, more preferably in the range of 0.25% to 80% by weight, still more preferably in the range of 0.5% to 70% by weight, still more preferably in the range of 0.5%-to 65% by weight, still more preferably in the range of 0.5%-to 50% by weight, still more preferably in the range of 0.5% to 20% by weight, and still more preferably in the range of 0.5% to 10% by weight, calculated to the total weight of the oxidative hair dyeing composition.

<14> The composition according to any of the clauses <12> to <13> characterized in that the oxidative hair dyeing composition comprises one or more pulverulent
excipients selected from organic/inorganic materials such as diatomaceous earth, cellulose, starch especially corn, tapioca, rice, wheat and potato, nylon powder, gypsum, sawdust and perlite.

<15> The composition according to clause <14> characterized in that the one or more pulverulent excipient is cornstarch.

<16> The composition according to any of the clauses <12> to <15> characterized in that the total concentration of the one or more pulverulent excipients is in the range of 1% to 80% by weight, preferably in the range of 2% to 75% by weight, more preferably in the range of 2.5% to 70% by weight, calculated to the total weight of the oxidative hair dyeing composition.

<17> The composition according to any of the clauses <12> to <16> characterized in that the total concentration of water in the oxidative hair dyeing composition is less than 5% by weight, more preferably less than 1% by weight, still more preferably less than 0.5% by weight, calculated to the total weight of the oxidative hair dyeing composition.

<18> The composition according to any of the clauses <12> to <16> characterized in that the oxidative hair dyeing composition is anhydrous.

<19> The composition according to any of the clauses <12> to <18> characterized in that the oxidative hair dyeing composition is a hair dyeing powder, a hair dyeing tablet, or a hair dyeing pellet.

<19> The composition according to any of the clauses <12> to <19> characterized in that the oxidative hair dyeing composition comprises one or more lipophilic compounds(s) being liquid at 25°C and atmospheric pressure.

<20> The composition according to clauses <19> characterized in that the one or more lipophilic compound(s) is/are isopropyl myristate, paraffin, squalane, phenyl trimethicone, and silicone, and/or their mixtures.

<21> The composition according to any of the clauses <19> to <20> characterized in that the total concentration of the lipophilic compound(s) is in the range of 0.1% to 20% by weight, more preferably in the range of 1% to 10% by weight, most preferably in the range of 2% to 6% by weight, calculated to the total weight of the oxidative hair dyeing composition.

<22> The composition according to any of the clauses <19> to <20> characterized in that the oxidative hair dyeing composition is a hair dyeing oil.

<23> The composition according to any of the clauses <19> to <20> and <22> characterized in that the total concentration of lipophilic compounds is in the range of 20% to 50% by weight, more preferably in the range of 25% to 40% by weight, most preferably in the range of 25% to 30% by weight, calculated to the total weight of the oxidative hair dyeing composition.

<24> The composition according to any of the clauses <12> to <23> characterized in that the oxidative hair dyeing composition comprises one or more alkalizing agent(s).

<25> The composition according to clause <24> characterized in that the one or more alkalizing agent(s) is/are one or more phosphate(s), one or more disilicate(s), one or more metasilicate(s), one or more carbonate(s), one or more bicarbonate(s), one or more Tris(hydroxymethyl)aminomethane, silicate(s), and/or its/their salts, and/or their mixtures, preferably their alkali and earth alkali salts.

<26> The composition according to clause <24> characterized in that the one or more alkalizing agent(s) is/are one or more phosphate(s) or one or more, metasilicate(s), and/or its/their salts, and/or their mixtures, more preferably the one or more alkalizing agent(s) are trisodium or tripotassium phosphate and sodium metasilicate, and/or their mixtures.

<27> The composition according to any of the clauses <24> to <26> characterized in that the total concentration of alkalizing agent(s) is in the range of 1% to 75% by weight, more preferably 5% to 70% by weight, still more preferably in the range of 10% to 70% by weight, still more preferably in the range of 15% to 70% by weight, calculated to the total weight of the oxidative dyeing composition.

<28> The composition according to any of the clauses <12> to <27> characterized in that the oxidative hair dyeing composition comprises one or more compound(s) according to group Z having the following general structure 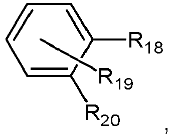 wherein R₁₈, R₁₉, and R₂₀ represent independently a hydrogen group, a hydroxyl group, an amino group, a sulfhydryl group, a methyl group, chlorine group, an acyl chloride group, C₁-C₄ alkoxy, C₁-C₄ hydroxyalkyl, preferably the one or more compound(s) according to group Z are selected from o-phenylenediamines, m-phenylenediamines, p-phenylenediamines, p-toluene-diamine, more preferably the one or more compound(s) according to group Z is p-phenylenediamine, 2-methoxy-methyl-p-phenylenediamine, p-toluene-diamine, hydroxyethyl-p-phenylene-diamine, and/or their salt(s), and/or their mixtures.

<29> The composition according to clause <28> characterized in that the one or more compound(s) according to group Z is 2-methoxy-methyl-p-phenylene-diamine, and/or its salt, preferably it is 2-methoxy-methyl-p-phenylene-diamine sulfate.

<30> The composition according to any of the clauses <28> to <29> characterized in that the total concentration of the one or more compound(s) according to group Z is in the range of 0.05% to 10% by weight, more preferably in the range of 0.1% to 7% by weight, most preferably in the range of 0.15% to 5% by weight, calculated to the total weight of the oxidative dyeing composition.

<31> The composition according to any of the clauses <12> to <30> characterized in that the oxidative dyeing composition comprises one or more compound(s) according to group Y selected from 4-chlororesorcinol, 1,3-bis(2,4-diamino-phenoxy)-propane, 2-methylresrocinol, 5-amino-6-chloro-o-cresol, and/or 2-amino-3-hydroxypyridine, and/or their salt(s), and/or their mixtures, and 2-methoxy-methyl-p-phenylene-diamine, and/or its salts as one or more compound(s) according to group Z.

<32> The composition according to any of the clauses <12> to <31> characterized in that the oxidative dyeing composition comprises a complex formed with thiamine hydrochloride as compound X and one or more compound(s) according to group Y selected from 4-chlororesorcinol, 1,3-bis(2,4-diamino-phenoxy)-propane, 2-methylresrocinol, 5-amino-6-chloro-o-cresol, and/or 2-amino-3-hydroxypyridine, and/or their salt(s), and/or their mixtures, and 2-methoxy-methyl-p-phenylene-diamine, and/or its salts as one or more compound(s) according to group Z.

<33> The composition according to any of the clauses <12> to <31> characterized in that the oxidative dyeing composition comprises a complex formed with thiamine as compound X and one or more compound(s) according to group Y selected from 4-chlororesorcinol, 1,3-bis(2,4-diamino-phenoxy)-propane, 2-methylresrocinol, 5-amino-6-chloro-o-cresol, and/or 2-amino-3-hydroxypyridine, and/or their salt(s), and/or their mixtures, and 2-methoxy-methyl-p-phenylene-diamine, and/or its salts as one or more compound(s) according to group Z.

<34> The composition according to any of the clauses <28> to <33> characterized in that the molar ratio of compound(s) according to group Y and the compound(s) according to group Z is in the range of 0.8 to 1.2, more preferably in the range of 0.9 to 1.1, most preferably it is 1.0, calculated to the free base of each of the compound(s).

<35> The composition according to any of the clauses <28> to <34> characterized in that the total concentration of one or more compound(s) according to group Y complexed by the one or more compound(s) according to group X, preferably said compound according to group X is thiamine or thiamine hydrochloride, in the oxidative hair dyeing composition is in the range of 0.1% to 90% by weight, preferably in the range of 0.25% to 80% by weight, more preferably in the range of 0.5% to 70% by weight, still more preferably in the range of 0.5% to 65% by weight, still more preferably in the range of 0.5% to 50% by weight, still more preferably in the range of 0.5% to 20% by weight, and still more preferably in the range of 0.5% to 10% by weight, calculated to the total weight of the oxidative hair dyeing composition.

The present disclosure is also directed to <36> a method of making the complex as defined in any of the clauses <1> to <11> comprising the steps of:
a) dissolving the one or more compounds X and Y in a solvent, preferably in water, followed by
b) evaporating the solvent and drying the complex.

<37> The method according to clause <36> characterized in that for evaporating in step b) heat is applied to the complex in the temperature range of 50°C to 100°C.

The present disclosure is also directed to <38> a kit-of-parts comprising:
- the oxidative hair dyeing composition as defined in any of the clauses <12> to <35>, and
- a second aqueous composition having a pH in the range of 1 to 6 and comprising one or more oxidizing agent(s), preferably hydrogen peroxide.

<39> The kit according to clause <38> characterized in that the total concentration of the hydrogen peroxide in the second aqueous composition is in the range of 1% to 20% by weight, more preferably in the range of 2% to 15% by weight, most preferably in the range of 3% to 12% by weight, calculated to the total weight of the second aqueous composition.

<40> The kit according to any of the clauses <38> to <39> characterized in that the pH of the second aqueous composition is in the range of 2 to 5, more preferably the pH is in the range of 2.5 to 4.5.

<41> The kit according to any of the clauses <38> to <40> characterized in that the oxidative dyeing composition as according to any of the claims <12> to <35> and the second aqueous composition are kept separate until directly prior to the application onto keratin fibers.

The present disclosure is also directed to <42> a method for dyeing keratin fibers, preferably human keratin fibers, more preferably human hair, comprising the steps of:
(i) mixing the oxidative dyeing composition as defined in any of the clauses <12> to <35> with a second aqueous composition as defined in any of the clauses <38> to <41> to yield a ready-to-use composition having a pH in the range of 7 to 12,
(ii) applying the ready-to-use composition onto keratin fibers and leaving it for a time period in the range of 1 min to 60 min,
(iii) rinsing-off the keratin fibers and optionally shampooing the keratin fibers.

<43> The method according to clause <42> characterized in that the pH of the ready-to-use composition in step (i) is in the range of 8 to 11, more preferably 8.5 to 10.5.

<44> The method according to any of the clauses <42> to <43> characterized in that the ready-to-use composition is left in step (ii) onto keratin fibers for a time period in the range of 5 to 45 min, more preferably 10 to 40 min.

The present disclosure is also directed to <45> a use of the compound X and/or its salt(s) according to any of the clauses <1> to <35> for improving the storage stability of the compound(s) Y and/or their salt(s) according to any of the clauses <1> to <35>.

The following examples are to illustrate the invention, but not to limit it.

### Examples

**Table 1 Inventive and comparative examples showing the effect of thiamine on storage stability.**

| **Ingredients** | | **Oxidative dyeing compositions** | | | | | | | |
|---|---|---|---|---|---|---|---|---|---|
| | | **Comp. Ex. 1** | **Comp. Ex. 2** | **Comp. Ex. 3** | **Comp. Ex. 4** | **Inv. Ex. 1** | **Inv. Ex. 2** | **Inv. Ex. 3** | **Inv. Ex. 4** |
| | | [% by weight] | | | | | | | |
| - | Trisodium phosphate | 65.70 | 65.70 | 65.70 | 65.70 | 65.70 | 65.70 | 65.70 | 65.70 |
| - | Behenyl alcohol* | 7.30 | 7.30 | 7.30 | 7.30 | 7.30 | 7.30 | 7.30 | 7.30 |
| - | NaHSO₄ | 9.00 | 9.00 | 9.00 | 9.00 | 9.00 | 9.00 | 9.00 | 9.00 |
| - | Phenyltrimethicone | 5.00 | 5.00 | 5.00 | 5.00 | 5.00 | 5.00 | 5.00 | 5.00 |
| - | Silica | 3.00 | 3.00 | 3.00 | 3.00 | 3.00 | 3.00 | 3.00 | 3.00 |
| (Z) | 2-methoxy-methyl-p-phenylene-diamine sulfate | 2.28 | 2.28 | 0.25 | 1.14 | 2.28 | 2.28 | 0.25 | 1.14 |
| (X) | Thiamine HCl | - | - | - | - | 3.10 | 3.07 | 0.34 | 1.53 |
| (Y) | 4-chlororesorcinol | 1.32 | - | - | - | 1.32 | - | - | - |
| | 2-methylresorcinol | - | 1.13 | - | - | - | 1.13 | - | - |
| | 1,3-bis(2,4-diamino-phenoxy)-propane 4HCl | - | - | 0.44 | - | - | - | 0.44 | - |
| | 2-amino-3-hydroxypyridine | - | - | - | 0.50 | - | - | - | 0.50 |
| - | Cornstarch | **Add to 100.00** | | | | | | | |
| **Evaluation - Storage Stability** | | | | | | | | | |
| Color Loss [ΔΔE] | | -8 | -14 | -5 | -7 | 0 | -2 | -1 | 0 |
| Water content [%] | | Less than 5% | | | | | | | |

| | | | | | | | | | |
|---|---|---|---|---|---|---|---|---|---|
| *for trisodium phosphate coating | | | | | | | | | |

**Table 2 Inventive examples.**

| **Ingredients** | **Oxidative dyeing compositions** | |
|---|---|---|
| | **Inv. Ex. 5** | **Inv. Ex. 6** |
| | **[% by weight]** | |
| Sodium metasilicate | 24.4 | 24.4 |
| Stearyl stearate** | 1.3 | 1.3 |
| Squalane | 5.0 | 5.0 |
| Silica | 3.0 | 3.0 |
| 2-methoxy-methyl-p-phenylene-diamine sulfate | 2.8 | 0.7 |
| 2-methylresorcinol (Y) | 1.4 | - |
| 5-amino-6-chloro-o-cresol (Y) | - | 0.4 |
| Thiamine HCl (X) | 3.8 | 0.9 |
| Cornstarch | **Add to 100.00** | |
| Water content [%] | Less than 5% | |

| | | |
|---|---|---|
| **for sodium metasilicate coating | | |

### Discussion of Results

The inventive compositions (s. Table 1, Inv. Ex. 1-4) comprising the thiamine(X)-coupler(Y)-complex were stored for 1 month at the temperature of 50°C and showed the highest stability juxtaposed to the comparative compositions (s. Table 1, Comp. Ex. 1-4) stored for 1 month at the temperature of 50°C. To evaluate the storage effect with and without the thiamine-coupler-complex the color loss was measured. The inventive compositions (Inv. Ex. 1-4) showed little or almost none change in the ΔE values (s. Table 1) for different couplers tested, whereas the comparative composition showed a strong decrease in dying intensity.

### Methods

### Thiamine-coupler-complex preparation for Table 1

The thiamine-coupler-complex was prepared by mixing thiamine HCl (X) with coupler (Y) in water in equimolar ratio (1:1). Then, the water was removed by vacuum evaporation procedure and the resulting product was dried at 70°C for 48 hours to harvest the coupler-thiamine complex. Then the complex was ground with mortar and pistil.

### Thiamine-coupler-complex preparation of Inv. Ex. 2-4 are prepared in the same manner as for Table 1.

### Preparation of Coated alkali (Trisodium phosphate coated by behenyl alcohol) in Table 1

To a 2L high-speed mixer (LFS-2 manufactured by Earthtechnica Co., Ltd., rotation number of the agitator 600 rpm, rotation number of the chopper 1500 rpm, jacket hot water temperature 80°C) was added 270 g of trisodium phosphate as the solid alkaline agent, powder temperature was confirmed to be 60°C or more, and 30 g of behenyl alchohol that had been melted as the coating material was added to the mixer, followed by mixing for 3 minutes to take mixture out. The mixture obtained was received on a tray and cooled at 25°C to obtain granules of the solid alkaline agent having the surface coated with the coating material.

### Preparation of Oxidative dyeing composition of Inv. Ex. 1-4 in Table 1

For the preparation of the oxidative dyeing composition, the sulfate salt of 2-methoxy-methyl-p-phenylenediamine (Z) was added in equimolar ratio with respect to the concentration of compounds Y to the thiamine-coupler-complex. Coated alkali, oil, liquidity improver, and acid were mixed to produce the composition. As a filler the cornstarch was added to the composition.

### Oxidative dyeing composition of Comp. Ex. 1-4 in Table 1 is the same manner as Inv. Ex. 1 except the complex.

### Preparation of Coated alkali (Sodium metasilicate coated by stearyl stearate) in Table 2

To a 2L high-speed mixer (LFS-2 manufactured by Earthtechnica Co., Ltd., rotation number of the agitator 600 rpm, rotation number of the chopper 1500 rpm, jacket hot water temperature 80°C) was added 290 g of Sodium metasilicate as the solid alkaline agent, powder temperature was confirmed to be 60°C or more, and 15.3 g of stearyl stearate that had been melted as the coating material was added to the mixer, followed by mixing for 3 minutes to take mixture out. The mixture obtained was received on a tray and cooled at 25°C to obtain granules of the solid alkaline agent having the surface coated with the coating material.

### Thiamine-coupler-complex preparation and preparation of oxidative dyeing composition in Table 2 was produced in the same manner as in Table1.

### Storage stability tests

To test the stability, the compositions (s. Table 1) were stored at 50°C for one month.

Goat Swatch hairstreaks (11 cm, 2 g per bundle) were shampooed and dried.

The dyeing compositions of table 1 were mixed prior to storage and after storage for 1 month with an oxidative composition comprising 6% by weight of hydrogen peroxide in a weight ratio of 1:10 to prepare ready-to-use compositions having a pH in the range of 9.5±0.5.

2 g of the ready-to-use compositions were applied onto the goat hairstreaks for 20 min at 40°C. The streaks were then rinsed off and dried. The colorimetric data were obtained with a color-difference meter (Datacolor Check II Plus) in the CIE colorimetric system (L*, a*, b*). Color differences (ΔE) were calculated by the following formula. $Δ E = \sqrt{{\left({L}_{1}^{∗}-{L}_{0}^{∗}\right)}^{2} + {\left({a}_{1}^{∗}-{a}_{0}^{∗}\right)}^{2} + {\left({b}_{1}^{∗}-{b}_{0}^{∗}\right)}^{2}}$ and $ΔΔE = {E}_{after storage} - {E}_{befor storage} .$

The water content of all samples was less than 5%.

## Claims

1. An oxidative hair dye complex comprising one or more compound(s) according to group X and one or more compound(s) according to group Y,
wherein the compound(s) according to group X have the following general structure: 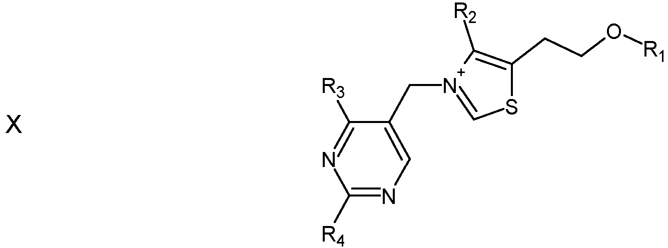
wherein R₁ represents a hydrogen atom, a phosphonate group, a pyrophosphate group, a triphosphate group, an adenosine diphosphate group, an adenosine triphosphate group, a linear or branched C₁-C₈ alkyl group, a linear or branched C₂-C₈ alkenyl group,
and wherein R₂, R₃, and R₄ represent independently a hydrogen, an amino group, linear or branched C₁-C₆ alkyl which may be substituted with one hydroxyl group, or linear or branched C₃-C₁₂ alkenyl which may be substituted with one hydroxyl group, wherein at least one of R₂, R₃, or R₄ is different from H,
and/or their salts, and/or their mixtures,
preferably the one or more compound(s) according to group X are selected from thiamine, thiamine hydrochloride, thiamine monophosphate, thiamine pyrophosphate, thiamine triphosphate, adenosine thiamine diphosphate, adenosine thiamine triphosphate, and/or their salt(s),
and wherein the compound(s) according to group Y are selected from the following general structures: 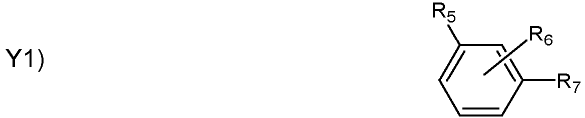
wherein R₅ and R₇ represent independently a hydroxyl group, an amino group, a sulfhydryl group, a linear or branched C₁-C₆ alkyl or alkenyl group, a halogen group, R₆ represents a hydrogen, a hydroxyl group, a halogen group, or a linear or branched C₁-C₆ alkyl or alkenyl group,
preferably the one or more compound(s) according to group Y1 are selected from resorcinol, 2-methylresorcinol, 4-chlororesorcinol, 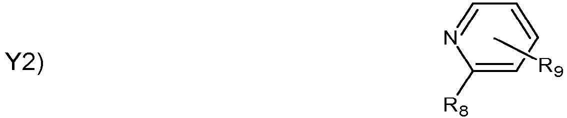
wherein R₈ and R₉ represent independently a hydroxyl group, an amino group, a sulfhydryl group, a linear or branched C₁-C₆ alkyl or alkenyl group, a halogen group,
preferably the one or more compound(s) according to group Y2 are selected from 2-amino-3-hydroxypyridine, and/or their salt(s), 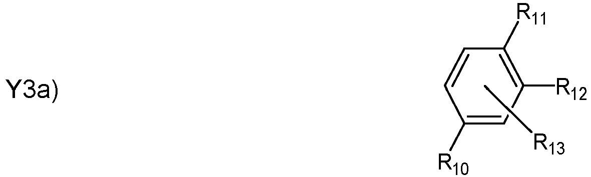 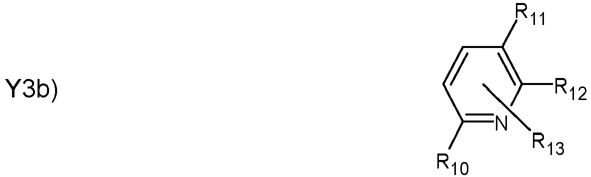
wherein R₁₀, R₁₁, R₁₂, and R₁₃ represent independently a hydrogen group, a hydroxyl group, an amino group, a methoxy group, a sulfhydryl group, a linear or branched C₁-C₆ alkyl or alkenyl group, a halogen group,
preferably the one or more compound(s) according to group Y3a or Y3b are selected from 5-amino-6-chloro-o-cresol, 2,6-dihydroxy-3,4-dimethyl-pyridine, 2,6-dimethoxy-3,5-pyridine-diamine HCl, 5-amino-2,6-dimethoxy-3-hydroxypyridine, 6-methoxy-2-methylamino-3-aminopyridine HCl, 2,3-diamino-6-methoxy-pyridine, 2,6-diaminopyridine, and/or their salt(s), 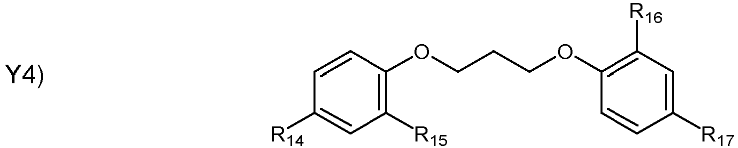
wherein R₁₄, R₁₅, R₁₆, and R₁₇ represent independently a hydroxyl group, an amino group, a sulfhydryl group, a linear or branched C₁-C₆ alkyl or alkenyl group, a halogen group,
and/or their salt(s), and/or their mixtures,
preferably the one or more compound(s) according to group Y4 are selected from 1,3-bis(2,4-diamino-phenoxy)-propane, and/or its salt(s).

2. The complex according to claim 1 **characterized in that** the molar ratio of one or more compound(s) according to group X to one or more compound(s) according to group Y is in the range of 0.8 to 1.2, preferably in the range of 0.9 to 1.1, more preferably 1.0, calculated to the free base of each of the compound(s).

3. An oxidative hair dyeing composition comprising the complex as defined in any of the claims 1 to 2, wherein the oxidative hair dyeing composition comprises water at 10% by weight or less, calculated to the total weight of the oxidative hair dyeing composition.

4. The composition according to claim 3 **characterized in that** the total concentration by weight of the complex in the oxidative hair dyeing composition is in the range of 0.1% to 90% by weight, preferably in the range of 0.25% to 80% by weight, more preferably in the range of 0.5% to 70% by weight, still more preferably in the range of 0.5% to 65% by weight, still more preferably in the range of 0.5% to 50% by weight, still more preferably in the range of 0.5% to 20% by weight, and still more preferably in the range of 0.5% to 10% by weight, calculated to the total weight of the oxidative hair dyeing composition.

5. The composition according to any of the claims 3 to 4 **characterized in that** the oxidative hair dyeing composition comprises one or more pulverulent excipients selected from organic/inorganic materials such as diatomaceous earth, cellulose, starch especially corn, tapioca, rice, wheat and potato, nylon powder, gypsum, sawdust and perlite, preferably said pulverulent excipient is cornstarch.

6. The composition according to claim 5 **characterized in that** the total concentration of one or more pulverulent excipients in the oxidative hair dyeing composition is in the range of 1% to 80% by weight, preferably in the range of 2% to 75% by weight, more preferably in the range of 2.5% to 70% by weight, still more preferably in the range of 5% to 50% by weight, still more preferably in the range of 5% to 30% by weight, calculated to the total weight of the oxidative hair dyeing composition.

7. The composition according to any of the claims 3 to 6 **characterized in that** the oxidative hair dyeing composition comprises one or more alkalizing agent(s), preferably selected from, phosphate(s), disilicate(s), metasilicate(s), carbonate(s), bicarbonate(s), Tris(hydroxymethyl)aminomethane, silicate(s), and/or its/their salts, and/or their mixtures.

8. The composition according to claim 7 **characterized in that** the total concentration of alkalizing agent(s) in the oxidative hair dyeing composition is in the range of 1% to 75% by weight, more preferably 5% to 70% by weight, still more preferably in the range of 10% to 70% by weight, still more preferably in the range of 15% to 70% by weight, calculated to the total weight of the oxidative hair dyeing composition.

9. The composition according to any of the claims 2 to 8 **characterized in that** the oxidative hair dyeing composition comprises one or more compound(s) according to group Z having the following general structure 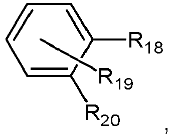 wherein R₁₈, R₁₉, and R₂₀ represent independently a hydrogen group, a hydroxyl group, an amino group, a sulfhydryl group, a methyl group, chlorine group, an acyl chloride group, C₁-C₄ alkoxy, C₁-C₄ hydroxyalkyl, preferably the one or more compound(s) according to group Z are selected from o-phenylenediamines, p-phenylenediamines, more preferably the one or more compound(s) according to group Z is p-phenylenediamine, 2-methoxy-methyl-p-phenylenediamine, p-toluene-diamine, hydroxyethyl-p-phenylene-diamine, and/or their salt(s), and/or their mixtures, and preferably the total concentration of one or more compound(s) according to group Z in the oxidative hair dyeing composition is in the range of 0.05% to 10% by weight, more preferably in the range of 0.1% to 7% by weight, most preferably in the range of 0.15% to 5% by weight, calculated to the total weight of the oxidative hair dyeing composition.

10. The composition according to any of the claims 3 to 9 **characterized in that** the one or more compound(s) according to group Y are selected from 4-chlororesorcinol, 1,3-bis(2,4-diaminophenoxy)-propane, 2-methylresrocinol, 5-amino-6-chloro-o-cresol, and/or 2-amino-3-hydroxypyridine, and/or their salt(s), and/or their mixtures.

11. The composition according to any of the claims 3 to 10 **characterized in that** the total concentration of water in the oxidative hair dyeing composition is 5% by weight or less, more preferably 1% by weight or less, still more preferably 0.5% by weight or less, calculated to the total weight of the oxidative hair dyeing composition, more preferably the oxidative hair dyeing composition is anhydrous.

12. A method of making the complex according to any of the claims 1 to 2 comprising the steps of:
a) dissolving the one or more compounds X and Y in a solvent, preferably in water, followed by
b) evaporating the solvent and drying the complex.

13. A kit-of-parts comprising:
- the oxidative hair dyeing composition as defined in any of the claims 3 to 11, and
- a second aqueous composition having a pH in the range of 1 to 6 and comprising one or more oxidizing agent(s), preferably hydrogen peroxide.

14. A method for dyeing keratin fibers, preferably human keratin fibers, more preferably human hair, comprising the steps of:
i) mixing the oxidative hair dyeing composition as defined in any of the claims 3 to 11 with a second aqueous composition as defined in claim 13 to yield a ready-to-use composition having a pH in the range of 6 to 12,
ii) applying the ready-to-use composition onto keratin fibers and leaving it for a time period in the range of 1 min to 60 min,
iii) rinsing-off the keratin fibers and optionally shampooing the keratin fibers.

15. Use of the compound X and/or its salt(s) as defined in any of the claims 1 to 11 for improving the storage stability of the compound(s) Y and/or their salt(s) as defined in any of the claims 1 to 11, wherein the oxidative hair dyeing composition comprises water at 10% by weight or less, calculated to the total weight of the oxidative hair dyeing composition.
